# EUROPÄISCHE PATENTSCHRIFT

(11) **EP 3 888 481 B1**
(45) Veröffentlichungstag und Bekanntmachung des Hinweises auf die Patenterteilung: **07.06.2023**
(21) Anmeldenummer: 20211823.8
(22) Anmeldetag: 04.12.2020
(51) Int. Cl.: A61B 5/00, A62B 18/02, G01B 11/25, A41D 13/11, A62B 23/02

(54) **VERFAHREN ZUM HERSTELLEN EINER BENUTZERINDIVIDUELLEN MUNDSCHUTZMASKE UND ENTSPRECHENDE SCHUTZMASKE**
METHOD OF MANUFACTURING A USER-INDIVIDUAL FACE MASK AND CORRESPONDING FACE MASK
MÉTHODE DE FABRICATION D'UN MASQUE FACIAL INDIVIDUEL ET MASQUE FACIAL CORRESPONDANT

(30) Priorität: 31.03.2020 DE 102020108870
(43) Veröffentlichungstag der Anmeldung: 06.10.2021
(73) Patentinhaber: senswork GmbH, 84489 Burghausen (DE)
(72) Erfinder: Obergrußberger, Rainer, 84568 Pleiskirchen (DE); Rieger, Roman, 83317 Teisendorf (DE); Gollwitzer, Jochen, 84570 Polling (DE)
(74) Vertreter: Lichtnecker, Markus Christoph

(56) Entgegenhaltungen:
- US-A1- 2008 006 273
- US-A1- 2014 278 319
- US-A1- 2015 352 382

## Beschreibung

Die Erfindung betrifft ein Verfahren zum Herstellen einer benutzerindividuellen Mundschutzmaske.

Mundschutzmasken sind grundsätzlich bekannt und werden z.B. als Schutzmasken oder Mundschutz bezeichnet.

Mundschutzmasken werden vor allem von medizinischem Personal getragen, um die Verbreitung von Krankheiten zu verhindern. Einerseits sollen Mundschutzmasken die Übertragung vom Träger der Mundschutzmaske auf weitere Personen verhindern. Andererseits soll das Ansteckungsrisiko des Trägers der Mundschutzmaske z.B. durch Patienten reduziert werden.

Gerade im Zusammenhang mit dem Coronavirus SARS-CoV-2 wurde die Bedeutung von Mundschutzmasken allgegenwärtig.

Während das Tragen von Mundschutzmasken im asiatischen Raum seit vielen Jahren bei Privatpersonen weit verbreitet ist, beispielsweise um bei einer Erkrankung keine anderen Personen anzustecken, werden Mundschutzmasken aufgrund der Corona-Pandemie mittlerweile auch in westlichen Ländern immer häufiger getragen.

Bei einem längeren Tragen von Mundschutzmasken, was gerade bei medizinischem Personal unvermeidbar ist, bilden sich am Gesicht jedoch unangenehme Druckstellen. Beispielsweise wird die Haut eingeschnürt und/oder eingedrückt, was zu Schmerzen und/oder Hämatomen führen kann.

US 2008/0006273 A1 und US 2015/0352382A1 offenbaren Verfahren gemäß dem Oberbegriff des Anspruchs 1.

In US 2014/0278319A1 werden Gesichtsdaten eines Benutzers erfasst und mit einer Datenbank, in der verschiedene Atemschutzmasken hinterlegt sind, abgeglichen.

Es ist eine Aufgabe der Erfindung, eine Mundschutzmaske sowie ein Verfahren zur Herstellung einer Mundschutzmaske zu schaffen, welche/s einen hohen Tragekomfort gewährleistet.

Die Lösung dieser Aufgabe erfolgt durch das Verfahren sowie den Gegenstand der unabhängigen Ansprüche.

Beim erfindungsgemäßen Verfahren wird eine benutzerindividuelle Mundschutzmaske hergestellt. Der Mundschutz wird demnach individuell für einen Benutzer hergestellt. Der hergestellte Mundschutz ist individuell an den Benutzer angepasst.

Bei dem Mundschutz handelt es sich insbesondere um keinen vollständigen Gesichtsschutz oder eine Beatmungsmaske. So wird beim Tragen insbesondere lediglich die untere Gesichtshälfte, insbesondere der Mund und/oder zumindest ein Abschnitt der Nase, abgedeckt.

Erfindungsgemäß werden Gesichtsdaten eines zu schützenden Gesichtsbereichs eines Benutzers erfasst. Dies kann insbesondere optisch erfolgen, beispielsweise mittels einer optischen Erfassungsvorrichtung. Auch mechanische Abdrücke sind denkbar.

Der zu schützende Gesichtsbereich umfasst insbesondere den Mund und/oder zumindest einen Abschnitt der Nase.

Vorzugsweise können weitere Gesichtsdaten, z.B. die Augen, erfasst werden.

Die Gesichtsdaten werden mit einer Datenbasis, in der anatomische Daten hinterlegt sind, abgeglichen. In der Datenbasis ist insbesondere eine große Anzahl an anatomischen Daten hinterlegt, beispielsweise von mindestens 100, 1.000, 10.000, 100.000, 1.000.000 oder deutlich mehr Personen.

Die anatomischen Daten können insbesondere Daten über die Position von Hart- und/oder Weichgewebe umfassen. Die Daten umfassen die Position von Knochen und/oder Knorpeln, z. B. des Kiefers, der Wangen und/oder der Nase. Vorzugsweise umfassen die Daten Informationen über eine untere, von außen nicht sichtbare Topologie.

Es wird ein dreidimensionales Modell des Gesichtsbereichs erstellt. Das 3D-Modell wird insbesondere anhand der erfassten und mit der Datenbasis abgeglichenen Gesichtsdaten erzeugt. Das 3D-Modell wird vorzugsweise lediglich virtuell erstellt. Alternativ oder zusätzlich kann ein physisches 3D-Modell erzeugt werden.

Anhand des Modells wird ein benutzerindividueller Kontaktrahmen hergestellt.

Dadurch ergibt sich ein hoher Tragekomfort für den Benutzer.

Der Kontaktrahmen kann insbesondere ringförmig ausgebildet sein. Grundsätzlich kann der Kontaktrahmen eine beliebige Form aufweisen. Die Breite des Kontaktrahmens kann beispielsweise zwischen 0,5 cm und 3 cm, insbesondere zwischen 1 cm und 2 cm, betragen.

Vorzugsweise umfasst der Kontaktrahmen ein weiches Material, z.B. ein, insbesondere thermoplastisches, Elastomer, ein Silikon und/oder einen PolyurethanSchaum.

Der Kontaktrahmen kann insbesondere tiefgezogen, aufgeschäumt, gegossen, zerspant, vorzugsweise ausgefräst, ausgeschliffen, ausgelasert und/oder ausgestrahlt sein. Auch kann der Kontaktrahmen im 3D-Druckverfahren hergestellt werden.

Der Kontaktrahmen steht im aufgesetzten Zustand mit dem Benutzer unmittelbar in Kontakt und berührt das Gesicht.

Insbesondere ist die Dicke des Kontaktrahmens nicht konstant. So kann die Dicke vorzugsweise variieren und beispielsweise an bestimmten Stellen, z.B. im Bereich der Nase, dicker oder dünner sein.

Die Dicke kann insbesondere zwischen 1 mm und 15 mm, vorzugsweise zwischen 3 mm und 10 mm, betragen.

Der Kontaktrahmen wird mit einer Funktionsvorrichtung luftdicht verbunden. Bei der Funktionsvorrichtung kann es sich insbesondere um ein Standardbauteil handeln, d.h. dieses ist nicht benutzerindividuell hergestellt. Alternativ kann die Funktionsvorrichtung benutzerindividuell hergestellt sein.

Die Funktionsvorrichtung umfasst wenigstens einen Filter zum Filtern von Partikeln, beispielsweise Feuchtigkeitspartikeln und/oder Feststoffpartikeln, aus der Luft. So handelt es sich bei der Mundschutzmaske gewissermaßen um eine Atemschutzmaske. Die Atemluft des Benutzers wird gefiltert.

Der Filter kann insbesondere als FFP1-, FFP2- oder FFP3-Filter ausgebildet sein. Vorzugsweise kann der Filter ein Textilmaterial, insbesondere ein Stoff- und/oder Vlies-Material, umfassen oder daraus bestehen.

Die Funktionsvorrichtung kann optional wenigstens ein Ventil aufweisen. Es können mehrere Ventile, z.B. zwei, drei, vier, fünf, sechs oder mehr Ventile, vorgesehen sein. Das Ein- und/oder Ausatmen wird dadurch erleichtert.

Die Mundschutzmaske kann insbesondere eine Kopfbefestigungsvorrichtung zur Befestigung mit dem Kopf eines Benutzers aufweisen. Beispielsweise kann die Kopfbefestigungsvorrichtung ein Band aufweisen, welches z.B. über den Hinterkopf gezogen wird. Auch können mehrere, insbesondere zwei, Bänder vorgesehen sein, welche z.B. an den Ohren des Benutzers befestigt werden. Alternativ ist eine Halterung zur Befestigung an einer Brille denkbar. Ferner kann ein Haftmaterial, insbesondere ein Klebematerial, vorgesehen sein, um die Mundschutzmaske am Gesicht des Benutzers festzukleben.

Die Kopfbefestigungsvorrichtung kann am Kontaktrahmen und/oder an der Funktionsvorrichtung befestigt sein.

Weiterbildungen der Erfindung sind auch den abhängigen Ansprüchen, der Beschreibung sowie den beigefügten Zeichnungen zu entnehmen.

Gemäß einer Ausführungsform werden dreidimensionale Gesichtsdaten erfasst, z.B. mittels einer optischen Erfassungsvorrichtung. Dabei kann es sich insbesondere um eine oder mehrere Kameras handeln. Beispielsweise kann eine Stereo- und/oder Multikamera vorgesehen sein. Ferner kann die Kamera eines Smartphones, Tablet-PCs, Desktop-PCs oder Laptops verwendet werden. Beispielsweise kann ein Bild oder mehrere Bilder bzw. ein Film aufgezeichnet werden. Dies kann der Benutzer insbesondere selbst, z.B. mit seinem Smartphone, vornehmen. Die Erfassung kann beispielsweise mittels Photogrammetrie erfolgen. Alternativ oder zusätzlich kann ein Scan, z.B. mittels eines Lasers oder mehrerer Laser, erfolgen.

Nach einer weiteren Ausführungsform werden die Gesichtsdaten mittels einer Streifenlicht-Projektion erfasst.

Dabei können Lichtstreifen auf den Gesichtsbereich projiziert werden. Eine Kamera, z.B. eine Stereokamera, kann, vorzugsweise beidseitig, die Krümmungen der Projektionslinien erfassen.

Eine Streifenlicht-Projektion liefert eine hohe Genauigkeit. Auch können die Gesichtsdaten schnell erfasst werden. Ferner können der Kopf sowie der Sensor bei der Erfassung stationär bleiben.

Gemäß einer weiteren Ausführungsform werden Gesichtsdaten bei geöffnetem und geschlossenem Mund erfasst. Von jedem Benutzer werden folglich vorzugsweise mindestens zwei Sätze an Gesichtsdaten erfasst. Insbesondere werden zwei unterschiedliche, dreidimensionale Modelle erstellt.

Der Kontaktrahmen kann derart hergestellt werden, dass dieser bestmöglich an beide Gesichtsdatensätze angepasst ist. So ist der Kontaktrahmen sowohl bei geöffnetem als auch bei geschlossenem Mund angenehm zu tragen.

Erfindungsgemäß weist die Datenbasis Datengruppen auf, in denen jeweils anatomische Daten einer Bevölkerungsgruppe hinterlegt sind.

Die Bevölkerungsgruppen weisen insbesondere anatomische Charakteristika auf. Insbesondere kann die Position und/oder Ausprägung bestimmter Knochen und/oder Knorpel bei verschiedenen Bevölkerungsgruppen unterschiedlich sein.

Bei den Bevölkerungsgruppen kann es sich beispielsweise um bestimmte Altersgruppen, z.B. Kinder, Jugendliche, Erwachsene, Senioren, handeln. Auch eine Unterteilung in männlich/weiblich/divers ist möglich. Daneben kann eine Unterteilung in ethnische Gruppe erfolgen. Ferner ist eine Unterteilung nach der Körpergröße und/oder dem Körpergewicht denkbar. Die Bevölkerungsgruppen können zudem in kombinierter Weise unterteilt werden.

Beispielsweise kann eine Bevölkerungsgruppe korpulente, große, männliche Erwachsene aus einer bestimmten Region der Erde umfassen. Die Wangenknochen müssen bei dieser Bevölkerungsgruppe durch den Kontaktrahmen beispielsweise weniger gepolstert werden, da diese unter einer dickeren Fettschicht als bei hageren Bevölkerungsgruppen liegt. Die Nase kann bei Personen aus einer bestimmten Region der Erde hingegen meist deutlich stärker ausgeprägt sein als bei Personen aus einer anderen Region der Erde, welche zum Teil kein Nasenbein besitzen. Folglich weist der Kontaktrahmen für eine Bevölkerungsgruppe A deutliche Unterschiede zu Kontaktrahmen für eine Bevölkerungsgruppe B auf.

In den Datengruppen können weitere anatomische Daten abseits des zu schützenden Gesichtsbereichs gespeichert sein, beispielsweise die Augen, z.B. die Augenfarben oder das Vorhandensein oder Abhandensein eines Augenlids.

Erfindungsgemäß umfassen die Daten einer Bevölkerungsgruppe Positionsdaten von Knochen, z.B. Wangen- und/oder Kieferknochen, und/oder Knorpeln. Die Knochen und/oder Knorpel können bei bestimmten Bevölkerungsgruppen anders positioniert bzw. stärker oder weniger stark ausgeprägt sein als bei anderen Bevölkerungsgruppen.

Nach einer weiteren Ausführungsform werden Charakteristika der Gesichtsdaten ermittelt und einer Datengruppe zugeordnet. Dies kann beispielsweise anhand des zu schützenden Gesichtsbereichs, z.B. der Nase und/oder des Mundes, erfolgen.

Auch weitere anatomische Charakteristika, z.B. die Augen, können alternativ oder zusätzlich herangezogen werden.

Die Zuordnung kann insbesondere computerbasiert, z.B. mittels Deep Learning und/oder Künstlicher Intelligenz, erfolgen.

Es ist ferner möglich, eine Zuordnung anhand weiterer personenbezogener Daten, z.B. des Alters, des Geschlechts, der Herkunft, der Körpergröße und/oder des Körpergewichts, zu erreichen bzw. zu verbessen.

Gemäß einer weiteren Ausführungsform wird das dreidimensionale Modell unter Berücksichtigung der anatomischen Daten der Datengruppe erstellt. Während die äußere Grundstruktur des zu schützenden Gesichtsbereichs optisch erfasst wird, können nichtsichtbare Merkmale, wie z.B. die Position der Knochen und/oder Knorpeln, anhand der der anatomischen Daten der entsprechenden Datengruppe bestimmt werden.

Der Kontaktrahmen kann dann anhand des dreidimensionalen Modells individuell hergestellt werden. Der Kontaktrahmen kann dann sowohl die äußere Struktur als auch die innere Struktur des zu schützenden Gesichtsbereichs berücksichtigen.

Nach einer weiteren Ausführungsform wird der Kontaktrahmen mit der Funktionsvorrichtung lösbar verbunden.

So kann beispielsweise die Funktionsvorrichtung ausgetauscht und der Kontaktrahmen wiederverwendet werden. Insbesondere kann der Kontaktrahmen gewaschen, desinfiziert und/oder sterilisiert werden.

Bei der Befestigungsvorrichtung zur Befestigung des Kontaktrahmens mit der Funktionsvorrichtung kann es sich insbesondere um eine Einhänge-, Klett-, Klammer-, Klemm-, Steck-, Rast-, Magnet-, Zapfen- und/oder Schraubvorrichtung handeln. Die Befestigung kann insbesondere werkzeuglos erfolgen. Ein schneller Austausch wird dadurch gewährleistet.

Auch unlösbare Verbindungen sind grundsätzlich denkbar, beispielsweise Klebe-, Niet- und/oder Schmelzverbindungen. Ferner können der Kontaktrahmen und Funktionsvorrichtung einstückig ausgebildet sein.

Die Erfindung betrifft schließlich eine Mundschutzmaske, die mit dem erfindungsgemäßen Verfahren hergestellt wird.

Alle hier beschriebenen Ausführungsformen und Bauteile der Vorrichtung sind insbesondere dazu ausgebildet, nach dem hier beschriebenen Verfahren hergestellt zu werden. Ferner können alle hier beschriebenen Ausführungsformen des Verfahrens sowie alle hier beschriebenen Ausführungsformen der Vorrichtung jeweils miteinander kombiniert werden, insbesondere auch losgelöst von der konkreten Ausgestaltung, in deren Zusammenhang sie erwähnt werden.

Die Erfindung wird im Folgenden beispielhaft unter Bezugnahme auf die Zeichnungen beschrieben. Es zeigen:
- Fig. 1: eine optische Erfassung von Gesichtsdaten, und
- Fig. 2: eine Perspektivansicht einer erfindungsgemäßen Mundschutzmaske.

Zunächst ist zu bemerken, dass die dargestellten Ausführungsformen rein beispielhafter Natur sind. So können einzelne Merkmale nicht nur in der gezeigten Kombination, sondern auch in Alleinstellung oder in anderen technisch sinnvollen Kombinationen realisiert sein. Beispielsweise können die Merkmale einer Ausführungsform beliebig mit Merkmalen einer anderen Ausführungsform kombiniert werden.

Enthält eine Figur ein Bezugszeichen, welches im unmittelbar zugehörigen Beschreibungstext nicht erläutert wird, so wird auf die entsprechenden vorhergehenden bzw. nachfolgenden Ausführungen in der Figurenbeschreibung Bezug genommen. So werden für gleiche bzw. vergleichbare Bauteile in den Figuren dieselben Bezugszeichen verwendet und diese nicht nochmals erläutert.

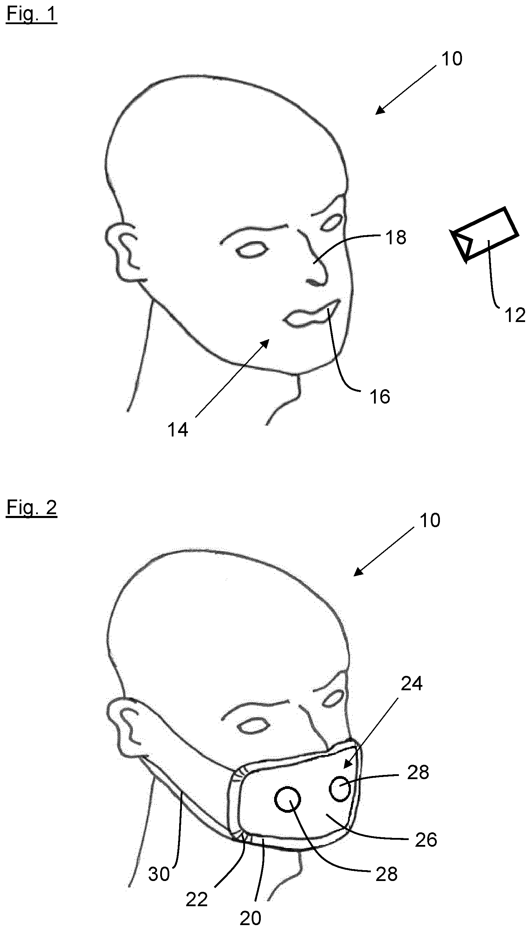

Fig. 1 zeigt einen Benutzer 10 sowie eine, als Kamera 12 ausgebildete optische Erfassungsvorrichtung.

Der zu schützende Gesichtsbereich 14 des Benutzers 10 umfasst den Mund 16 und zumindest einen Abschnitt der Nase 18.

Mit der optischen Erfassungsvorrichtung 12 werden dreidimensionale Gesichtsdaten erfasst.

Insbesondere können zwei Datensätze - bei offenem und geschlossenem Mund - erfasst werden.

Die Gesichtsdaten werden optional mit einer Datenbasis, in der anatomische Daten hinterlegt sind, abgeglichen. Die Datenbasis umfasst vorzugsweise Datengruppen, in denen jeweils anatomische Daten, z.B. die Ausprägung und/oder die Positionsdaten von hartem Gewebe, Weichgewebe, Knochen und/oder Knorpeln, einer Bevölkerungsgruppe hinterlegt sind.

Anschließend können Charakteristika der Gesichtsdaten, z.B. die Größe und/oder Form der Nase, ermittelt und einer Datengruppe zugeordnet werden.

Insbesondere unter Berücksichtigung der anatomischen Daten der Datengruppe wird ein dreidimensionales Modell erstellt. In das Modell fließen sowohl die unmittelbar optisch erfasste äußere Struktur, vorzugsweise bei geöffnetem sowie geschlossenen Mund, als auch vorzugsweise die mittels der Datenbasis ermittelte innere Struktur des zu schützenden Gesichtsbereichs ein. Die innere Struktur, insbesondere die Position der Knochen und/oder Knorpeln, kann alternativ oder zusätzlich metrisch bestimmt werden.

Anhand des Modells wird ein in Fig. 2 gezeigter, benutzerindividueller Kontaktrahmen 20 hergestellt. Der Kontaktrahmen 20 kann unterschiedlich dick sein, um sich an den zu schützenden Gesichtsbereich 14 optimal anzupassen.

Ferner kann der Kontaktrahmen 20 einen oder mehrere Falzbereiche 22 aufweisen, um auch beim Öffnen bzw. Schließen des Mundes 16 einen optimalen Sitz zu ermöglichen. Die Falzbereiche 22 können z.B. ziehharmonikaförmig ausgebildet sein.

Der Kontaktrahmen 20 wird luftdicht mit einer Funktionsvorrichtung 24 verbunden. Die Funktionsvorrichtung 24 weist einen Filter 26 auf. Optional können ein oder mehrere Ventile 28 vorgesehen sein.

Mittels einer, zwei Bänder 30 umfassenden Kopfbefestigungsvorrichtung kann die Mundschutzmaske schließlich am Kopf des Benutzers 10 befestigt werden.

### Bezugszeichenliste

- 10: Benutzer
- 12: Kamera, optische Erfassungsvorrichtung
- 14: zu schützender Gesichtsbereich
- 16: Mund
- 18: Nase
- 20: Kontaktrahmen
- 22: Falzbereich
- 24: Funktionsvorrichtung
- 26: Filter
- 28: Ventil
- 30: Band, Kopfbefestigungsvorrichtung

## Patentansprüche

1. Verfahren zum Herstellen einer benutzerindividuellen Mundschutzmaske, bei dem,
insbesondere optisch, Gesichtsdaten eines zu schützenden Gesichtsbereichs (14) eines Benutzers (10) erfasst werden,
ein dreidimensionales Modell des Gesichtsbereichs (14) erstellt wird, und anhand des Modells ein benutzerindividueller Kontaktrahmen (20) hergestellt wird, wobei
der Kontaktrahmen (20) der Mundschutzmaske mit einer Funktionsvorrichtung (24) luftdicht verbunden wird, wobei
die Funktionsvorrichtung (24) wenigstens einen Filter (26) zum Filtern von Partikeln aus der Luft umfasst,
**dadurch gekennzeichnet,**
**dass** die Gesichtsdaten nach dem Erfassen mit einer Datenbasis, in der anatomische Daten hinterlegt sind, abgeglichen werden,
wobei die Datenbasis Datengruppen aufweist, in denen jeweils anatomische Daten einer Bevölkerungsgruppe hinterlegt sind,
wobei die Daten einer Bevölkerungsgruppe Positionsdaten von Knochen und/oder Knorpeln umfassen.

2. Verfahren nach Anspruch 1,
**dadurch gekennzeichnet,**
**dass** dreidimensionale Gesichtsdaten erfasst werden.

3. Verfahren nach Anspruch 1 oder 2,
**dadurch gekennzeichnet,**
**dass** die Gesichtsdaten mittels einer Streifenlicht-Projektion erfasst werden.

4. Verfahren nach einem der vorhergehenden Ansprüche,
**dadurch gekennzeichnet,**
**dass** Gesichtsdaten bei geöffnetem und geschlossenem Mund (16) erfasst werden.

5. Verfahren nach einem der vorhergehenden Ansprüche,
**dadurch gekennzeichnet,**
**dass** die Daten Informationen über eine untere, von außen nicht sichtbare Topologie umfassen.

6. Verfahren nach einem der vorhergehenden Ansprüche,
**dadurch gekennzeichnet,**
**dass** die Daten einer Bevölkerungsgruppe Positionsdaten des Kiefers, der Wangen und/oder der Nase, umfassen.

7. Verfahren nach einem der vorhergehenden Ansprüche,
**dadurch gekennzeichnet,**
**dass** eine Unterteilung der Bevölkerungsgruppen nach Altersgruppen, männlich/weiblich/divers, ethnischen Gruppen, der Körpergröße und/oder dem Körpergewicht erfolgt.

8. Verfahren nach einem der vorhergehenden Ansprüche,,
**dadurch gekennzeichnet,**
**dass** Charakteristika der Gesichtsdaten ermittelt werden und einer Datengruppe zugeordnet werden.

9. Verfahren nach einem der vorhergehenden Ansprüche,
**dadurch gekennzeichnet,**
**dass** in den Datengruppen weitere anatomische Daten abseits des zu schützenden Gesichtsbereichs gespeichert sind.

10. Verfahren nach einem der vorhergehenden Ansprüche,
**dadurch gekennzeichnet,**
**dass** der Kontaktrahmen (20) mit der Funktionsvorrichtung (24) lösbar verbunden wird.

11. Mundschutzmaske, **dadurch gekennzeichnet, dass** sie mit dem Verfahren nach einem der vorhergehenden Ansprüche hergestellt ist.

## Claims

1. Method for producing a user-individual mouth protection mask,
in which method,
in particular optically, facial data of a user (10) facial region (14) to be protected are captured,
a three-dimensional model of the facial region (14) is created, and a user-individual contact frame (20) is produced on the basis of the model, wherein
the contact frame (20) of the mouth protection mask is connected in an airtight manner to a functional device (24), wherein
the functional device (24) comprises at least one filter (26) for filtering particles from the air,
**characterised in that**
the facial data, after being captured, are matched with a database in which anatomical data are stored, wherein
the database has data groups, in each of which anatomical data of a population group are stored, wherein
the data of a population group comprise positional data of bones and/or cartilages.

2. Method according to claim 1,
**characterised in that**
three-dimensional facial data are captured.

3. Method according to claim 1 or 2,
**characterised in that**
the facial data are captured by means of a fringe projection technique.

4. Method according to any of the preceding claims,
**characterised in that**
facial data are captured with the mouth (16) open and closed.

5. Method according to any of the preceding claims,
**characterised in that**
the data comprise information about a lower topology that is not visible from the outside.

6. Method according to any of the preceding claims,
**characterised in that**
the data of a population group comprise positional data of the jaw, the cheeks and/or the nose.

7. Method according to any of the preceding claims,
**characterised in that**
a subdivision of the population groups is made according to age groups, male/female/diverse, ethnic groups, body height and/or body weight.

8. Method according to any of the preceding claims,
**characterised in that**
characteristics of the facial data are determined and assigned to a data group.

9. Method according to any of the preceding claims,
**characterised in that**
further anatomical data other than the facial region to be protected are stored in the data groups.

10. Method according to any of the preceding claims,
**characterised in that**
the contact frame (20) is detachably connected to the functional device (24).

11. A mouth protection mask,
**characterised in that**
it is produced by the method of any of the preceding claims.

## Revendications

1. Procédé de fabrication d'un masque de protection buccale spécifique à l'utilisateur, dans lequel
des données de visage d'une zone de visage (14) à protéger d'un utilisateur (10) sont saisies, en particulier par voie optique,
un modèle tridimensionnel de la zone de visage (14) est créé, et un cadre de contact (20) spécifique à l'utilisateur est fabriqué à l'aide du modèle,
le cadre de contact (20) du masque de protection buccale est relié de manière étanche à l'air à un dispositif fonctionnel (24),
le dispositif fonctionnel (24) comprend au moins un filtre (26) pour filtrer les particules de l'air,
**caractérisé en ce que**
une fois saisies, les données de visage sont comparées à une base de données dans laquelle sont stockées des données anatomiques,
la base de données comprend des groupes de données dans chacun desquels sont stockées les données anatomiques d'un groupe de population,
les données d'un groupe de population comprennent des données de position des os et/ou des cartilages.

2. Procédé selon la revendication 1,
**caractérisé en ce que**
des données de visage tridimensionnelles sont saisies.

3. Procédé selon la revendication 1 ou 2,
**caractérisé en ce que**
les données de visage sont saisies au moyen d'une projection de lumière en bande.

4. Procédé selon l'une des revendications précédentes,
**caractérisé en ce que**
les données de visage sont saisies lorsque la bouche (16) est ouverte et fermée.

5. Procédé selon l'une des revendications précédentes,
**caractérisé en ce que**
les données comprennent des informations sur une topologie inférieure non visible de l'extérieur.

6. Procédé selon l'une des revendications précédentes,
**caractérisé en ce que**
les données d'un groupe de population comprennent des données de position de la mâchoire, des joues et/ou du nez.

7. Procédé selon l'une des revendications précédentes,
**caractérisé en ce que**
une subdivision des groupes de population se fait selon les groupes d'âge, le sexe masculin/féminin/divers, les groupes ethniques, la taille et/ou le poids du corps.

8. Procédé selon l'une des revendications précédentes,
**caractérisé en ce que**
des caractéristiques des données de visage sont déterminées et attribuées à un groupe de données.

9. Procédé selon l'une des revendications précédentes,
**caractérisé en ce que**
d'autres données anatomiques, en dehors de la zone de visage à protéger, sont mémorisées dans les groupes de données.

10. Procédé selon l'une des revendications précédentes,
**caractérisé en ce que**
le cadre de contact (20) est relié de manière amovible au dispositif fonctionnel (24).

11. Masque de protection buccale,
**caractérisé en ce que**
il est fabriqué par le procédé selon l'une des revendications précédentes.
